# EUROPEAN PATENT APPLICATION

(11) **EP 1 788 079 A1**
(43) Date of publication of application: **23.05.2007**
(21) Application number: 05765782.7
(22) Date of filing: 08.07.2005
(51) Int. Cl.: C12N 5/06

(54) **ANIMAL TISSUE-ECCENTRICALLY LOCATED PLURIPOTENT CELL PROLIFERATING SELECTIVELY IN LOW-SERIUM MEDIUM**

(30) Priority: 08.07.2004 JP 2004201615
(71) Applicant: Japan Science and Technology Agency, Kawaguchi-shi, Saitama 332-0012 (JP); KYOWA HAKKO KOGYO CO., LTD., Chiyoda-ku Tokyo 100-0004 (JP); Kitagawa, Yasuo, B4, Gurandomezon-Hikarigaoka 57-21, Fujimidai 5-chome, Chikusa-ku Nagoya-shi, Aichi 464-0015 (JP); Torii, Shuhei, 2-5-2, Nishiyamamotomachi, Chikusa-ku Nagoya-shi, Aichi 464-0063 (JP)
(72) Inventor: SAKURADA, Kazuhiro, Yokohama-shi, Kanagawa 225-0016 (JP); KOBORI, Masato, Sagamihara-shi, Kanagawa 229-0004 (JP); KAMEI, Yuzuru, Nagoya-shi, Aichi 466-0051 (JP); YAMAGUCHI, Hirotake, Nagoya-shi, Aichi 467-0054 (JP); TORIYAMA, Kazuhiro, Nagoya-shi, Aichi 466-0022 (JP); TAKADA, Toru, Nagoya-shi, Aichi 460-0012 (JP)
(74) Representative: Gardner, Rebecca Katherine
(86) International application number: PCT/JP2005/013100
(87) International publication number: WO 2006/006692

(57) **Abstract**

It is intended to provide a pluripotent cell having the following properties: (1) being contained in a mixed-cell type population obtained by enzymatic treatment of the tissue collected from an animal; (2) being contained in a sedimented cell population obtained by centrifugating the mixed-cell type population of the property (1); (3) selectively proliferating by culturing in a medium containing 2%(v/v) or lower serum and 1 to 100 ng/ml of fibroblast growth factor-2; and (4) being differentiated into cells having the characteristics of adipocytes, osteoblasts, chondrocytes, tendon cells, myocardial cells, myoblasts, neurocytes or vascular endothelial cells by adjusting the culture condition; cells having differentiated from the above cell; and a method of conveniently obtaining the pluripotent cell in a large amount.

## Description

### Technical Field

The present invention relates to pluripotent cells that exist ubiquitously in animal tissues, proliferate by culturing *in vitro,* differentiate into tissue cells of the animal body, and can replace the body tissues suffering from disorders or dysfunctions by transplanting the same. The invention also relates to a method for obtaining the pluripotent cells.

### Background Art

In the place of healthcare depending on "medicaments", the era of regenerative medicine has started, in which a complex system of the human body is generated in large quantities outside the human body and replaced with a degenerated complex system suffering from disorders or dysfunctions. In the future, the "medical materials" may take over the leading role of "medicaments", and the industries producing "medical materials" may rival with the pharmaceutical industries in such a day. In this situation, the most widely noticed matter among the "medical materials" is a "pluripotent cell" (or "stem cell"). Recently, highly proliferative and pluripotential stem cells have been discovered in many countries, and technological development such as isolating and proliferating the stem cells from the human body and forming the partial structure of human body from the stem cells has actively been promoted. These pluripotent cells can be classified into embryonic stem cell (master cell) differentiating into all sorts of human body cells, hemotopoietic stem cell producing blood cells, and neural stem cell utilizable in therapy of central nervous system disorders such as Parkinson's disease. Mesenchymal stem cells are also regarded as important since they differentiate into mesenchymal tissue cells such as bone constituting the human body framework, cartilage, skeletal muscle, myocardium, smooth muscle, and fat. If such pluripotent cells can be separated conveniently and safely from patients to proliferate in vitro in large quantities, it would be expected that they can bring about essential revolution in the medical field which reinforce, repair, substitute and reconstruct lacked or hypofunction body.

In order to treat diseases caused by abnormality in hemocytes such as leukemia, hypoplastic anemia and lymphoma, a method of transplanting bone marrow, in which a cell population containing hemocyte stem cells collected from the bone marrow of a healthy volunteer is transplanted to a patient, has been established. In this bone marrow transplantation, an official system designated as Bone Marrow Donor Program has been developed to avoid rejection due to histocompatibility, wherein a donor whose histocompatibility matches that of the patient is chosen from a large number of registered donors so that the transplantation can be achieved not only among a descent group but also between persons with no blood relation. In this bone marrow transplantation, the collected bone marrow aspirate is directly transplanted to the patient without any processing such as in vitro culture for the growth of the hematopoietic stem cells.

A cultured skin has been developed in order to use in treatment of serious burning, chronic dermal ulcer of diabetic patients, pressure sore in aged persons, or nevus pigmentosus. The skin includes cultured dermis and cultured epidermis, and in both cases the cells used are merely differentiated into fibroblast-like cells or epidermal keratinocytes, indicating that pluripotential stem cells are not utilized. In addition, transplantation between individuals in which histo compatibility is not recognized for purpose of using in emergency medical care is also considered.

It has been discovered that the pluripotent cells having an ability to differentiate into the cells of various body tissues exist in a bone marrow aspirate, and a method for separation thereof has been developed. The content of the pluripotent cells in the bone marrow aspirate, however, is no more than 0.01 to 0.001% for the total cells, and in order to secure 10⁸ or more cells necessary for regenerative therapy, 100 liters to 1000 liters of bone marrow aspirate is required. Though the researchers have attempted a treatment of autotransplanting the pluripotent cells of this type which are separated selectively from the bone marrow aspirate of the patient directly without any cultural operation, the collection of a large quantity of bone marrow aspirate imposes a severe burden to the patient with danger.

The present inventors have reported that pluripotential stem cells (pluripotent cells) are inherent in adipose tissues (Non-patent Document 1). Marc H. Hedrick et al. (University of California, Los Angels) have reported that they centrifuged a large quantity of adipose tissue collected from fat patients to separate the pluripotent cells by suction to separate the pluripotent cells and found the cell population of sedimentation fractions (SVF fraction) containing the pluripotent cells (Non-patent Document 2). In addition, it has also been reported that satellite cells which are known to exist in the muscle are pluripotential stem cells (Non-patent Document 3).

The content of these pluripotent cells, however, is very low, resulting in very low yield of the pluripotent cells. In addition, the SVF fraction is an extremely heterogeneous cell population which makes it unclear which cell contained in the SVF fraction is a pluripotent cell. In fact, Hedrick et al. succeeded first in separation of the mesenchymal stem cells using 330 ml of aspirated fats (Non-patent Document 2); this is, however, insufficient in practical use since there is few fat patients from whom a large quantity of fats can be extracted. In order to obtain the pluripotent cells from muscle, a single colony separation method is necessary, in which much time and labor are required.

In this connection, as for the prior art relative to acquisition of the pluripotent cells, there are Non-Patent Documents 2 and 3 in addition to the present inventor's patent application (Patent Document 1).

### List of Documents

Patent Document 1: JP-A-2004-129549
Patent Document 2: JP-A-2002-052365
Patent Document 3: International Publication WO99/27076 pamphlet
Non-patent Document 1: Kawaguchi et al., Proc Natl Acad Sci USA. 1998; 95:1062-1066
Non-patent Document 2: Zuk et al., Tissue Engineering, 2001; 7, 211-228
Non-patent Document 3: Wada et al., Development, 2002; 129, 2987-2995

### Disclosure of Invention

For the lacked or dysfunctioned bone, cartilage, skeletal muscle, myocardium, fat and nerve, a treatment for reinforcement, repairing, replacement and reconstruction has been conducted by transplantation of the pluripotent cells collected from the patient's self or another person whose histocompatibility matches that of the patient. In this treatment, however, the pluripotent cells are required at a rate of 10⁸ cells or more per case.

In the prior art, however, a large quantity of bone marrow aspirate or adipose tissues is required in order to separate such type of pluripotent cells (particularly, mesenchymal stem cells), and further much time and labor are required for expanding the culture volume of pluripotent cells. In order for such a treatment of transplanting the pluripotent cells to become popular, it is necessary to specify a pluripotent cell which can be collected conveniently and safely, by means of an efficient method for separation thereof and a technology for allowing proliferation thereof.

The present invention was made in view of the above circumstances for a purpose of providing a pluripotent cell (particularly, pluripotent cell having an ability to differentiate into adipocytes, osteoblasts, chondrocytes, tendon cells, myocardial cells, myoblast, neurocytes or vascular endothelial cells) collected conveniently, safely and in large quantities, and a cell differentiated from the pluripotent cell.

In addition, a purpose of the invention is to provide a method for obtaining the above-mentioned pluripotent cells.

Further, a purpose of the invention is to provide a method for transplanting a cell using the above-mentioned pluripotent cells.

The first invention for achieving the above purpose is a pluripotent cell having the following properties:
(1) the cell is contained in a mixed-cell type population obtained by enzymatic treatment of the tissue collected from an animal;
(2) the cell is contained in a sedimented cell population obtained by centrifugating the mixed-cell type population of the property (1);
(3) the cell selectively proliferates by culturing in a medium containing 2%(v/v) or lower serum and 1 to100 ng/ml of fibroblast growth factor-2; and
(4) the cell is differentiated into cells having the characteristics of adipocytes, osteoblasts, chondrocytes, tendon cells, myocardial cells, myoblasts, neurocytes or vascular endothelial cells by adjusting the culture condition.

In a preferred embodiment of the first invention, the animal includes, preferably, human, monkey, mouse, rat, bovine, equine, pig, dog, cat, goat, sheep or chicken; the collected tissue includes subcutaneous adipose, , greater omentum, visceral adipose, muscle or organ.

In another preferred embodiment of the first invention, the cells included in the mixed-cell type population of the property (1) are CD34-positive and CD45-negative cells; and the selectively proliferating cells of the property (3) are CD34-negative, CD13-positive, CD90-positive and CD105-positive cells.

The 2nd invention is a cell differentiated from the pluripotent cell of the first invention, which has any of the characteristics of adipocytes, osteoblasts, chondrocytes, tendon cells, myocardial cells, myoblast, neurocytes or vascular endothelial cells.

The 3rd invention is a method for obtaining a pluripotent cell having characteristics of adipocytes, osteoblasts, chondrocytes, tendon cells, myocardial cells, myoblast, neurocytes or vascular endothelial cells, which comprises:
(a) a step for preparing a mixed-cell type population by treating an animal tissue with an enzyme;
(b) a step for preparing a sedimented cell population by centrifuging the mixed-cell type population of the step (a); and
(c) a step for selecting a cell selectively proliferating from the cell population of the step (b) by culturing in a medium containing 2%(v/v) or lower serum and 1 to 100 ng/ml of fibroblast growth factor-2.

In a preferred embodiment of the 3rd invention, the tissue of human, monkey, mouse, rat, bovine, equine, pig, dog, cat, goat, sheep or chicken is treated with an enzyme to prepare the mixed-cell type population in the step (a), and further subcutaneous adipose, greater omentum, visceral adipose, muscle or organ in an animal is treated with an enzyme to prepare the mixed-cell type population in the step (a).

In a preferred embodiment of the 3rd invention, a CD34-positive and CD45-negative cell-type population is prepared in the step (a), and a CD34-negative, CD13-positive, CD90-positive and CD105-positive cell is selected in the step (b).

In addition, the 4th invention is a method for cell-transplantation which comprises transplanting the pluripotent cell of the 1st invention into the animal body.

In the above invention of this application, "pluripotent cells" refer to the cells (stem cells) which are capable of differentiating to the cells having the characteristics of bone, cartilage, skeletal muscle, myocardium, fat, tendon, ligament, interstitial cell, neurocyte or vascular endothelial cell. In the following explanation, in some cases, the term pluripotent cell is described merely as "stem cell".

### Brief Description of Drawings

Fig.1 shows a growth curve of pluripotent cell derived from the subcutaneous adipose of a 22-year-old man.
Fig.2 shows growth curves of the pluripotent cells in the presence of fetal calf serum or human serum.
Fig.3 shows a photograph indicating that the proliferated pluripotent cells derived from the subcutaneous adipose of a 22-year-old man was differentiated into adipocyte.
Fig.4 shows a photograph indicating that the proliferated pluripotent cell derived from the subcutaneous adipose of a 22-year-old man was differentiated into osteoblast.
Fig.5 shows a photograph indicating that the proliferated pluripotent cell derived from the greater omentum tissue of a 48-year-old woman was differentiated into adipocyte.
Fig.6 shows a photograph indicating that the proliferated pluripotent cell derived from the greater omentum tissue of a 48-year-old woman was differentiated into osteoblast.
Fig.7 shows a photograph indicating that the proliferated pluripotent cell derived from the subcutaneous adipose of a 22-year-old man was differentiated into chondrocyte.
Fig.8 shows a photograph indicating that the proliferated pluripotent cell derived from a CD34-positive and CD45-negative cell population have a characteristic of differentiated fat.
Fig.9 shows a photograph indicating that the proliferated pluripotent cell derived from a CD34-positive and CD45-negative cell population have a characteristic of differentiated osteoblast.
Fig.10 shows a photograph showing fat regeneration when the proliferated pluripotent cell derived from the subcutaneous adipose of a 22-year-old man was transplanted into a mouse.

### Effect of the Invention

According to the present invention, it makes it possible to obtain pluripotent cells of 10⁸ cells or more, in practice, 10⁹ cells or more, which is necessary in transplantation for reconstruction of tissues without any burden to a donor. It also makes it possible to proliferate selectively the pluripotent cells required for transplantation. The pluripotent cells can be transplanted into the body of animal (donor itself or other recipient) to form adipocytes, bone, cartilage, muscle, nerve, blood vessel, and the like. Thus, according to the present invention, the donor's own pluripotent cells differentiatable to a variety of tissues can be provided in large quantities, and accordingly, the transplantation of such cells into the donor's body allows a treatment for reconstruction of the lacked or hypofunctioned bone, cartilage, adipose tissues or the like. In treatment of a patient who lost the soft tissue due to trauma or cancer or a patient suffering from facial hemiatrophy in which the subcutaneous connective tissue on the half side of the face is contracted, the patient's own adipocytes are auto-transplanted to the necessary part; this operation, however, had disadvantages that it had a tendency to cause absorption or scar after the transplantation to lose the volume of transplantation. The reason is considered to be necrosis of matured adipocytes occupying most of the transplanted fat, but if the stem cells selectively proliferated in vitro can be transplanted, the problem is expected to be solved. Such a reconstruction technique of mesenchymal tissues is also expected to develop into the field of cosmetic surgery such as breast enlargement operation. By practically using the pluripotency of pluripotent cells, it is possible to reconstruct the skeleton on large scale such as skelton lost by open fracture. If the pluripotent cells are confirmed to differentiate into myocardial cells, some heart diseases which attack high and middle aged groups in the prime of life might be expected to be cured. The method for collecting the donor's pluripotent cells successfully provided by the invention will lead to development of such a new area of regeneration medicine; thus, it will exert an immeasurable influence. This system allowing the mass-production of pluripotent cells from a safely and conveniently available fat can be expanded to healthy people, resulting in establishment of a "pluripotent cell bank"; thus, transplantation from young people to aged generation is expected between the histocompatible individuals.

### Best Mode for Carrying Out the Invention

The pluripotent cells of the first invention are characterized in that they have the above properties (1) to (4) and can be prepared by the method of the 3rd invention. That is, the method of the 3rd invention can be carried out in the following procedure.

### (a) Step for preparing a mixed-cell type population

The animal tissue includes, for example, those of human, monkey, mouse, rat, bovine, equine, pig, dog, cat, goat, sheep or chicken. The tissue can be isolated from the animal body by resection, including adipose tissues such as subcutaneous adipose, greater omentum, visceral adipose surrounding mesentery or kidney, epididymis adipose, and intramuscular tissue adipose, as well as muscle, heart, lung, liver, kidney, stomach, small intestine, large intestine, etc. More preferably, subcutaneous adipose and greater omentum are included. The amount of the tissues to be collected varies with the species of donor and the type of tissues, and usually it is for example in about 1 to 10 g, and particularly in case of human donor, preferably about 2g considering the burden to the donor.

In case of adipose tissues, for example, the subcutaneous adipose is collected by aspiration from a donor or a small quantity (e.g., spindle-form of 1 cm width, 2 cm length and 0.5 cm in depth) of skin and subcutaneous adipose is collected from the body surface of a donor. In case of greater omentum, a small quantity of greater omentum is collected peritoneally from a generally anesthetized donor with an endoscope.

The resulting animal tissue piece is washed, for example, with a culture medium to remove blood or the like.

Enzymatic treatment may be carried out by digesting these animal tissues with an enzyme such as collagenase, trypsin, pronase, dispase, elastase or hyaluronidase. Such treatment with an enzyme can be achieved according to the procedure and condition well known to persons skilled in the art (see: R.I. Freshney, Culture of Animal Cells: A Manual of Basic Technique, 4th Edition, A John Wiley & Sons Inc., Publication). Alternatively, the treatment may be carried out according to the procedure and condition as described in Examples below.

The mixed-cell type population can be obtained by such enzymatic treatment. This mixed-cell type population contains two or more species of heterologous cells, for example, mesenchymal stem cells, tissue cell populations at various mature stages, endothelial cells, pericytes, interstitial cells, and various blood cells.

The mixed-cell type population may also be prepared as a CD34-positive and CD45-negative cell population, wherein the type of expressive antigen is utilized as an index. As shown in Examples below, since the cells converted into some pluripotent cells in the end are CD34-positive and CD45-negative in the cell type popuration, the preparation of such surface marker-printing cell population allows to obtain pluripotent cells more efficiently. The CD34-positive and CD45-negative cells can be separated and collected by conventional means well known in the art using the respective antibodies (i.e., anti-CD34 antibody, anti-CD45 antibody) or using micro-beads to which these antibodies are bound.

### (b) Step of preparing a sedimented cell population

The mixed-cell type population prepared in the step (a) can be centrifuged to give a sedimented cell population. That is, the enzyme-untreated tissues contained in the mixed-cell type population are removed by filtration, and the resulting cell suspension is applied to centrifugation. The centrifugation may be conducted, for example, at 800 to 1500 rpm for a period of 1 to 10 minutes, though variable depending on the characters and amount of the cells. Thus, the sedimentation cell population can be obtained as sediment after centrifugation.

Thus resulting sedimented cell population contains, for example, mesenchymal stem cells, tissue cell population at various mature stages, endothelial cells, pericytes, interstitial cells, and various blood cells.

### (c) Step of selective culture on a low-serum medium

In this step, the cell population as mentioned in the step (b) is cultured on a culture medium containing 2%(v/v) or lower serum and 1 to 100 ng/ml of fibroblast growth factor-2 (FGF-2) to select a selectively proliferating cell.

As for the culture medium, a conventional medium for animal cell culture may be used, provided that the serum content therein is 2%(v/v) or less. The culture medium includes, for example, Alpha-MEM (Dai-Nippon Pharmaceutical Co., etc.), ATCC-CRCM 30 (ATCC), Coon's modified F12 (SIGMA, etc.), DM-160 and DM-201 (Nihon Seiyaku KK), Dulbecco's modified Eagle's Medium (DMEM) with high glucose (4500 mg/L)(Dai-Nippon Pharmaceutical Co., etc.), Dulbecco's modified Eagle's Medium (DMEM) with low glucose (1000 mg/L)(Wako Pure Chemical Ind.), DMEM:Ham's F12 mixed medium (1:1)(Dai-Nippon Pharmaceutical Co., etc.), DMEM:RPMI1640 mixed medium (1:1), Eagle's basal medium (EBM)(Dai-Nippon Pharmaceutical Co., etc.), Eagle's Minimum Essential Medium (EMEM)(Dai-Nippon Pharmaceutical Co., etc.), EMEM:RPMI1640 mixed medium (1:1), ES medium (Nissui Seiyaku KK), Fischer's medium (Wako Pure Chemical Ind., etc.), Ham's F10 (Dai-Nippon Pharmaceutical Co., etc.), Ham's F12 medium (Dai-Nippon Pharmaceutical Co., etc.), Ham's F12:RPMI1640 mixed medium (1:1), Kaighns modification of Ham's F12 (F12K)(Dai-Nippon Pharmaceutical Co., etc.), Leibovitz's L-15 medium (Dai-Nippon Pharmaceutical Co., etc.), McCoy's 5A (Dai-Nippon Pharmaceutical Co., etc.), RITC80-7 medium (Research Institute for the Functional Peptides), HF-C1 medium (Research Institute for the Functional Peptides), MCDB107 medium (Research Institute for the Functional Peptides), MCDB201 medium (SIGMA), HSMC-C1 medium (Research Institute for the Functional Peptides), HEC-C1 medium (Research Institute for the Functional Peptides), MCDB131 medium (Research Institute for the Functional Peptides), HSMC-C2 medium (Research Institute for the Functional Peptides), MCDB153 medium (Research Institute for the Functional Peptides), MCDB153HAA medium (Research Institute for the Functional Peptides), Medium 199 (Dai-Nippon Pharmaceutical Co., etc.), NCTC135 (Dai-Nippon Pharmaceutical Co., etc.), RPMI1640 (Dai-Nippon Pharmaceutical Co., etc.), Waymouth's MB752/1 medium (Dai-Nippon Pharmaceutical Co., etc.), William's medium E (Dai-Nippon Pharmaceutical Co., etc.), and the like.

When these media are serum-free, 2%(v/v) or lower serum is added thereto. And when a serum-containing medium is used, the content of serum is adjusted to 2%(v/v) or lower by a method for removing the serum and so on. In a usual culture method of animal cells, fetal calf serum is used in a concentration of 10 to 20%(v/v), but in the method of this invention the serum content is reduced to 2%(v/v) or lower in order to restrict the activity of cell growth factors contained in the serum as an indicated culture condition. The "serum" is not limited to fetal calf serum for use in a usual method but includes human serum which can be taken out from the patients.

Thus, the pluripotent cells of the first invention can be proliferated selectively by culturing in such a medium. The stem cells grown in the above culture condition can be applied to subculture to give 10⁸ or more of pluripotent cells required for one cycle of tissue regeneration medicine, since they have a high proliferation activity.

Alternatively, the selectively proliferating cell may also be prepared as a CD34-negative, CD13-positive, CD90-positive and CD105-positive cell, wherein the type of cell surface antigen is utilized as an index. As shown in Examples mentioned below, since the cells converted into some pluripotent cells in the end have a cell surface antigen as mentioned above, the pluripotent cells can be obtained more efficiently by obtaining such cell surface antigen-presenting cell. The CD34-negative, CD13-positive, CD90-positive and CD105-positive cells can be separated and collected by conventional means well known in the art using the respective antibodies or using micro-beads to which these antibodies are bound.

The pluripotent cells (cells of the first invention) obtained in the above method (the method of the 3rd invention) are differentiated into cells which have characteristics of adipocytes, osteoblasts, chondrocytes, tendon cells, myocardial cells, myoblast, neurocytes or vascular endothelial cells by adjusting the culture condition.

For example, the differentiation into adipocytes can be carried out by culturing the cells on an adipocyte-inducing medium containing a suitable amount of fetal calf serum, isobutyl-1-methylxanthine, indometacin, hydrocortisone, insulin, dexamethasone, and the like, as shown in Example 4 described below. The differentiation into osteoblasts may be carried out by culturing the cells on an osteoblast-inducing medium containing a suitable amount of fetal calf serum, ascorbic acid phosphate ester magnesium salt n-hydrate, β-glycerophosphate, dexamethansone, and the like, as shown in Example 5 described below. Further, the differentiation into chondrocytes may be carried out, for example, using a chondrocyte-inducing medium prepared by adding 100 ng/ml of TGF-β1, and the like, as shown in Example 7 described below. The differentiation into vascular endothelial cells may be induced in a medium containing a vascular endothelial cell growth factor, the differentiation into myoblasts may be induced in a medium containing a suitable amount of 5-azacytidine, the differentiation into the neurocytes may be induced in a medium containing a suitable amount of 2-mercaptoethanol or dimethylsulfoxide, the differentiation into myocardial cell may be induced in medium containing a suitable amount of 5-azacytidine, and the differentiation into the tendon cells may be induced by culturing in collagen gel with suitable physical irritation, respectively.

The cell of the 2nd invention is a differentiated cell having any of the characteristics of adipocytes, osteoblasts, chondrocytes, tendon cells, myocardial cells, myoblast, neurocytes or vascular endothelial cells. In order to make the cell differentiate into a particular cell efficiently, the mixed-cell type population prepared from a particular animal tissue respectively may preferably be used to obtain the pluripotent cell as mentioned in the method of the 3rd invention. That is, when the pluripotent cells are differentiated into adipocytes, the mixed-cell type population may be prepared from adipose tissue; for the differentiation into osteoblast or chondrocyte, the cell population is prepared from bone tissue; for differentiation into myoblast, from muscular tissue; for differentiation to nerve cell, from nerve tissue; and for differentiation into vascular endothelial cell, from vascular tissue, respectively.

The 4th invention is a method for transplanting cells, which comprises transplanting the pluripotent cells of the first invention into the animal body. That is, as will be shown in Examples, when the pluripotent cells of the first invention are transplanted into the animal body, the cells are differentiated into another cells that have the same characteristics as the parent cells of the tissue. Therefore, the pluripotent cells can be transplanted to a variety of mammals including human; for example, it is possible to treat an animal (human, or non-human animal including domestic animals and pet animals), in which a particular function is damaged due to the loss or disappearance of particular functional cells, for recovering said cell function. In addition, a particular cell can be transplanted into a non-human mammal to make the cell grow excessively therein, and thus an animal model in which a particular cell function is enhanced can be generated (e.g., obesity model of animal having excessive adipocytes).

The transplantation of the pluripotent cells may be carried out, for example, according to Examples as described below. The number of cells to be transplanted, though variable depending on the species of cells or animals, may be 10⁶ or more. When human or non-human animal is a recipient, it is preferable to transplant the pluripotent cells prepared from the same individual. In case of a non-human animal such as animal model, the donor and the recipient may be different individual by using an animal lacking the immune function.

### Examples

The following examples will explain the invention of this application specifically in more detail, but the invention of this application is not limited by the examples.

### Example 1

### Preparation of a low serum medium suitable to culture of the stem cells contained in adipose tissues

In the primary culture of the cells, a low serum medium was used, which was prepared by adding 1/100 part of linoleic acid-albumin (SIGMA) and 100×ITS supplement (SIGMA), 0.1 mmol/L of ascorbic acid phosphate ester magnesium salt n hydrate (Wako Pure Chemical Ind.), 50 U/ml of penicillin (Meiji Seika Kaisha, Ltd.), and 50 µg/ml of streptomycin (Meiji Seika Kaisha, Ltd.) to a 3:2 mixture of Dulbecco's modified Eagle's medium (Nissui Pharmaceutical Co., Ltd.) and MCDB201 medium (SIGMA) to give a serum-free basal medium, then adding 2%(v/v) fetal calf serum (ICN Biomedical Inc.) thereto, and further adding 20 ng/ml of human FGF-2 (PeproTech EC Inc.).

### Example 2

### Preparation of adipose tissue-derived stem cells by low serum culture

From a 22-year-old male patient with his informed consent, subcutaneous adipose tissue (1.2 g) at the back normal part which was a residue of operation was recovered. This was washed with a equal volume mixture of Dulbecco's modified Eagle's medium and Ham's F12 medium (Nissui Pharmaceutical Co., Ltd.)(DMEM/F12 medium) to remove adhering blood and others. The adipose tissue was cut with a pair of surgical scissors into square pieces of 2 mm in size, to which was added 2.4 ml of 1 mg/ml collagenase solution (collagenase type I, WORTHINGTON), and the mixture was shaken at 37°C for 1 hour. Thus treated solution was filtered through a steel mesh (250 µm pore size) to remove tissue pieces not digested with collagenase. The cell suspension was centrifuged at 1200 rpm at room temperature for 5 minutes to give a sedimented cell population as precipitate (SVF fraction). The SVF fraction was washed 3 times with DMEM/F12 medium by centrifugation and stained by a Türk's solution (Nacalai Tesque, Inc.) to count the number of nucleated cells.
The cells (1.6×10⁵) were inoculated in a 25 cm² flask (NUNC) coated with human fibronectin (SIGMA), to which was added 5 ml of low serum medium containing 2%(v/v) fetal calf serum, and the mixture was incubated at 37°C in 5% CO₂/95% air saturated condition. Twenty four hours after the inoculation in the flask, the cells not adhering to the bottom of flask such as erythrocytes were removed together with the medium, and the fresh low serum medium was added.

### Example 3

### Subculture of the stem cells selected by low serum culture

The cells grown immediately before a confluent state in Example 2 was washed with 1 mmol/L EDTA (Wako Pure Chemical Industries Inc.)/phosphate buffered physiological saline (Nissui Pharmaceutical Co., Ltd.), to which was added 0.25%(w/v) trypsin solution (SIGMA), and the mixture was incubated for 2 minutes so that the cells were peeled off. Fresh low serum medium was added, and the cells were dispersed therein and stained by a Türk's solution to count the number of cells. The cells (2×10⁵) were inoculated in a fresh 25 cm² flask coated with human fibronectin and cultured at 37°C in 5% CO₂/95% air saturated condition. The culture medium was changed with fresh low serum medium every 2 days.

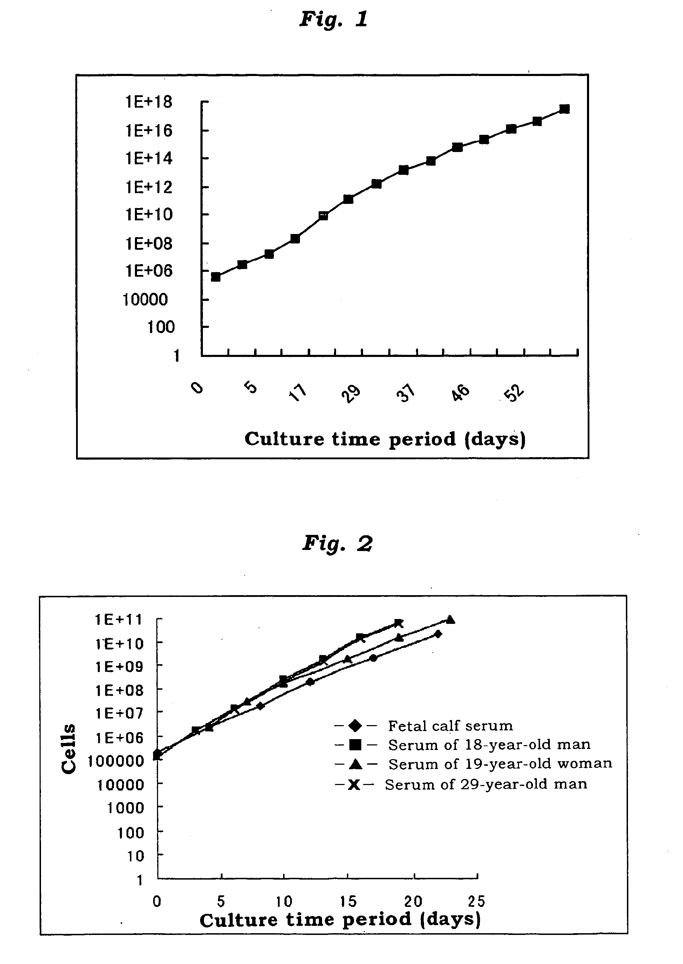

Fig.1 shows the results. That is, after 13 subcultures over 52 days, the cells were counted to proliferate up to 10¹⁸ cells (Fig.1). This high proliferation ability indicates that these cells have on average of 40 cycles or more of division potential.
In the case of Fig.1, the subculture was made on a medium containing 2%(v/v) fetal calf serum; alternatively, the same or much better effect was attained using a culture medium containing 2%(v/v) human serum (CosmoBio Co., Ltd). Fig.2 shows growth curves of the pluripotent cells derived from the subcutaneous adipose tissue of a man of 22 years old, which cells were cultured on a low serum medium containing 2%(v/v) human serum derived from a man of 18 years old, a woman of 19 years old or a man of 29 years old, respectively.

### Example 4

### Induction to adipocytes

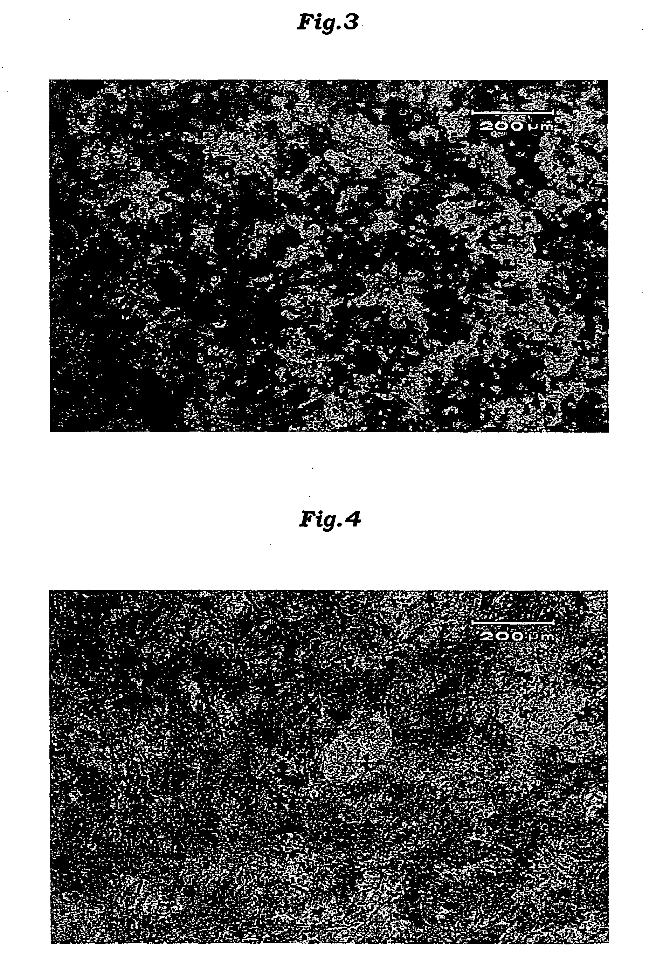

The cells in which division and proliferation were repeated at an average of 10 times were cultured in a 25 cm² flask coated with human fibronectin, and immediately before a confluent state, an adipocyte-inducing medium (Dulbecco's modified Eagle's medium, 10%(v/v) fetal calf serum, 0.5 mmol/L isobutyl-1-methylxanthine (SIGMA), 0.1 mmol/L indomethacin (Wako Pure Chemical Industries Ltd.), 1 µmol/L hydrocortisone (SIGMA), 10 µg/ml insulin (SIGMA), and 1 µmol/L dexamethasone (SIGMA)) was added, and the mixture was cultured at 37°C in 5% CO₂/95% air saturated condition for 15 days. The culture medium was changed with fresh one every 3 days. Morphological change specific to the adipocytes was observed by an inverted microscope. The characteristics of adipocytes containing oily drops were observed in 90% or more cells (Fig.3).

### Example 5

### Induction to osteoblasts

The cells in which division and proliferation were repeated at an average of 10 times were cultured in a 25 cm² flask coated with human fibronectin on a DMEM/F12/20%(v/v) fetal calf serum medium, and immediately before a confluent state, an osteoblast-inducing medium (Dulbecco's modified Eagle's medium, 10%(v/v) fetal calf serum, 50 µmol/L ascorbic acid phosphate ester magnesium salt n-hydrate, 10 mmol/L β-glycerophosphate (SIGMA), and 0.1 µmol/L dexamethasone) was added, and the mixture was cultured at 37°C in 5% CO₂/95% air saturated condition for 3 weeks. The culture medium was changed with fresh one in every 3 days.

### Example 6

### Alkaline phosphatase reaction and von Kossa staining (Confirmation of differentiation into osteoblast)

After 3 weeks from the beginning of induction, the culture medium was removed, and the cells were washed once with a phosphate buffered physiological saline. The cells were immersed in 10%(v/v) neutrally buffered formaldehyde solution (Wako Pure Chemical Industries Ltd.) for 15 minutes for fixation, then washed once with distilled water, and further immersed in distilled water for 15 minutes. An alkaline phosphatase substrate solution (0.2 mg/ml Naphtol AS MX-PO4 (SIGMA), 0.8%(v/v) N,N-dimethylformamide (Wako Pure Chemical Ind.), 1.2 mg/ml Fast Red Violet LB salt (SIGMA), and 0.1 mol/L Tris hydrochloric acid buffer solution (pH 8.3)) was added, and the mixture was allowed to react at room temperature for 45 minutes, and then washed 3 times with distilled water. Then, 2.5%(w/v) silver nitrate aqueous solution (Wako Pure Chemical Ind.) was added, and the mixture was allowed to react at room temperature for 30 minutes, and washed 3 times with distilled water. Fig.4 shows the state of staining.

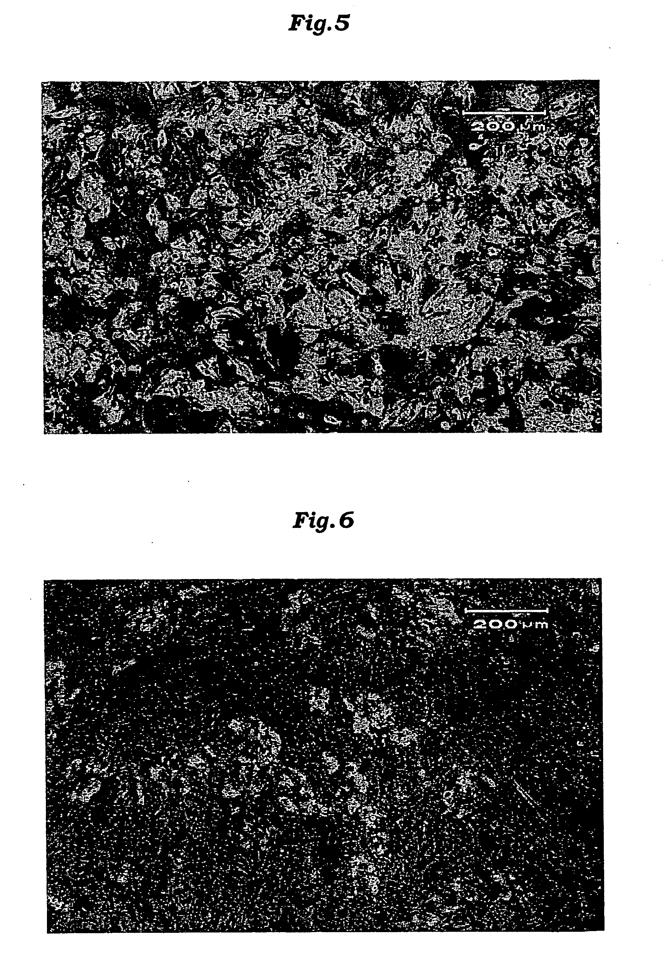

As shown in Fig.4, it was confirmed that the pluripotent cells of the invention, when subjected to differentiation induction to osteoblasts, can be differentiated into osteoblasts in high efficiency.
In addition, the human greater omentum tissue could be treated in the same manner as in Examples 1 to 3 to give a fibroblast-like cell, which could be applied to repetition of subculture and had a high proliferation rate and pluripotency for differentiating into fat and osteocyte. Figures show the induction to adipocytes by the method as shown in Example 4 (Fig.5) or the induction to osteoblast by the method as shown in Examples 5 and 6 (Fig.6), in both cases the induction was made from the stem cell derived from the epiploon tissue of a 48-year-old woman by average 20 repetitions of division and proliferation. The stem cells which have a pluripotency and are differentiated into adipocytes or osteoblasts were also recovered from epiploon tissue.

### Example 7

### Induction to chondrocyte

The cells in which division and proliferation were repeated at an average of 10 times were cultured in a 25·cm² flask coated with human fibronectin, and immediately before a confluent state, a chondrocyte-inducing medium (serum-free basal medium to which 100 ng/ml of TGF-β1 (PeproTech Inc.) was added) was added, and the mixture was cultured at 37°C in 5% CO₂/95% air saturated condition for 12 days. The culture medium was changed with fresh one every 4 days. Morphological change specific to the chondrocytes was observed by an inverted microscope. The morphological change and aggregation of the cells were observed TGF-β1 dependently.

### Example 8

### Alcian Blue staining

### (Confirmation of differentiation into chondrocyte)

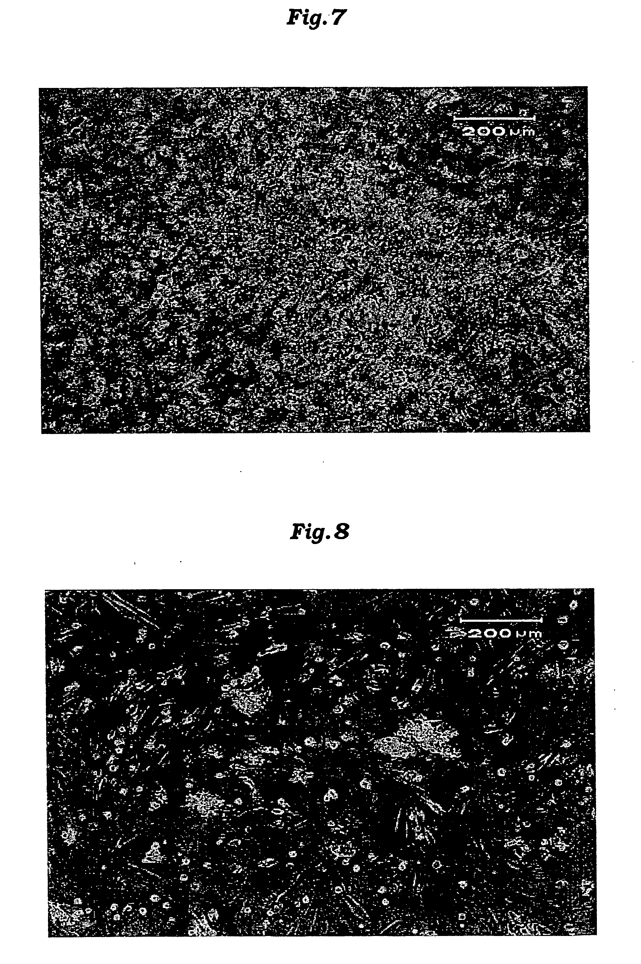

After 12 days from the beginning of induction, the culture medium was removed, and the cells were washed once with a phosphate buffered physiological saline. The cells were immersed in 10%(v/v) neutrally buffered formaldehyde solution for 15 minutes for fixation, then washed once with distilled water, and further immersed in 0.1 mol/L of hydrochloric acid solution for 5 minutes. A staining solution of 1%(w/v) Alcian Blue (SIGMA) dissolved in 0.1 mol/L of hydrochloric acid was added, and the mixture was allowed to react at room temperature for 30 minutes. The cells were washed once with 0.1 mol/L of hydrochloric acid and then with distilled water. The state of staining is shown in Fig.7. The cells which were induced to differentiation TGF-β1 dependently were stained to greenish blue, indicating that the cells were positive to chondroitin sulfate being chondrocyte-specific glucosaminoglucan.

### Example 9

### Analysis of cell surface antigen of uncultured enzyme-treated SVF fraction by a flow cytometer

The cells (10⁴) of the SVF fraction derived from human subcutaneous adipose tissue recovered from a 12-year-old man according to the method as described in Example 2 were stained by PE-labeled anti-human CD34 antibody and PC7-labeled anti-human CD45 antibody (both from Beckman Coulter Inc.) and analyzed by flow cytometry. Both antibodies were diluted to 1/20 with phosphate buffered physiological saline containing 0.1%(w/v) bovine serum albumin (Nacalai Tesque, Inc.) and incubated at 4°C for 30 minutes. After incubation, the cells were washed with phosphate buffered physiological saline and analyzed by a JSAN desktop cell sorter (Bay Bioscience Co., Ltd). About 50% of uncultured SVF fraction was a CD34-positive and CD45-negative cell population, and about 40% was a CD34-negative and CD45-positive cell population.
Further, these cell population were stained by anti-human CD13, CD90, CD105 antibodies (all from Beckman Coulter Inc.) and anti-human CD37 antibody (Becton, Dickinson and Co.), and analyzed by flow cytometry. The CD34-positive and CD45-negative cell population contained in the uncultured SVF fraction were positive to CD37 and CD90 and negative to CD13 and CD105.

### Example 10

### Identification of a cell population in the SVF fraction selectively proliferating by low serum culture

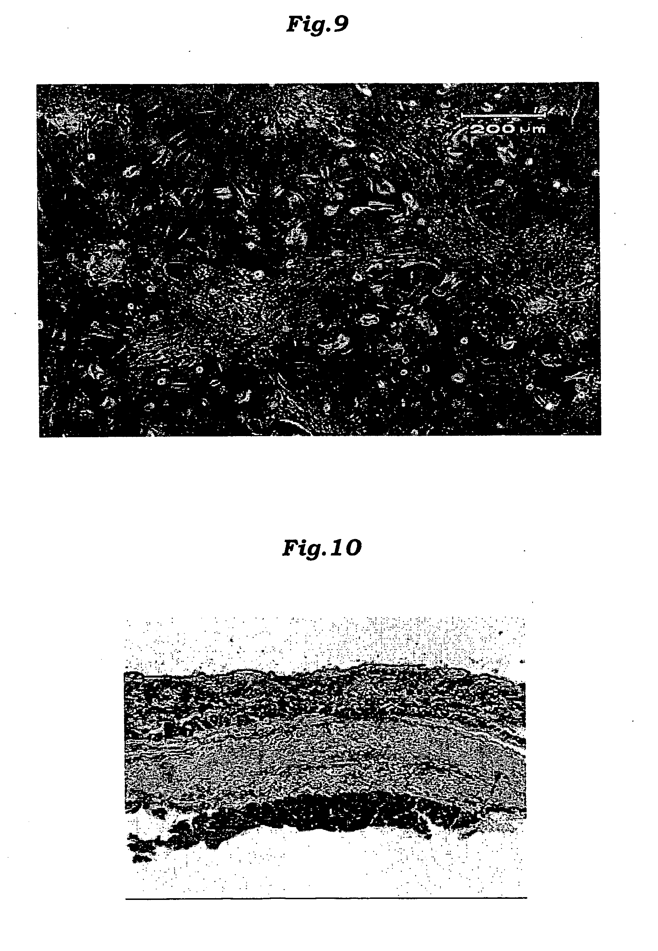

The SVF fraction derived from human greater omentum tissue recovered from a 49-year-old woman according to the method as described in Example 2 was separated into a CD45-negative cell population and a CD45-positive cell population using CD45 microbeads (Daiichi Pure Chemicals Co., Ltd). The CD45-nagative cell population was further separated to a CD34-positive cell population and a CD34-negative cell population using CD34 microbeads (Daiichi Pure Chemicals Co., Ltd).
Thus separated CD45-positive cell population, CD34-positive and CD45-negative cell population, and CD34-negative and CD45-negative cell population were respectively cultured in a low serum medium as shown in Example 1. The cells derived from the CD45-positive cell population were not generally grown; the cells derived from the CD34-negative and CD45-negative cell population were moderately grown; and the cells derived from the CD34-positive and CD45-negative cell population were well grown. Thus grown cells derived from CD34-negative and CD45-negative cell population, and the cells derived from the CD34-positive and CD45-negative cell population were respectively induced to differentiate into adipocytes and osteoblasts by the method as described in Examples 4 and 5, respectively. As a result, the cells derived from the CD34-positive and CD45-negative cell population only exhibited characteristics of adipocytes (Fig.8) and osteoblasts (Fig.9).

### Example 11

### Analysis of the cell surface antigen of the cell cultured in a low serum medium by a flow cytometer

The SVF fraction derived from human subcutaneous adipose tissue obtained according to the method as described in Example 2 was grown immediately before a confluent state on a low serum medium in the same manner as in Example 3, and the cells divided at an average of 10 times were peeled off and dispersed. The cells (10⁴) were respectively stained by an anti-human CD13, CD31, CD34, CD45, CD90, CD105, CD106, or CD117 antibody (all from Beckman Coulter Inc.), and analyzed by flow cytometry. The cell population proliferating from the SVF fraction by low serum culture was positive to CD13, CD90 and CD105, and negative to CD31, CD34, CD45, CD106 and CD117.
According to the method as described in Example 10, the CD34-positive and CD45-negative cell population separated from the SVF fraction were grown immediately before a confluent state by low serum culture, and the cells divided at an average of 10 times were peeled off and dispersed. The cells (10⁴) were respectively stained by an anti-human CD13, CD31, CD34, CD45, CD90, CD105, CD106, or CD117 antibody, and analyzed by flow cytometry. The cell population proliferating from the CD34-positive and DC-45 negative cell population in the SVF fraction by low serum culture was positive to CD13, CD90 and CD 105, and negative to CD31, CD34, CD45, CD 106 and CD 117. The cell population proliferating from the whole cell population of SVF fraction by low serum culture exhibited the same expression manner of cell surface antigen as the cell population proliferating from the CD34-positive and CD45-negative cell population only in the SVF fraction by low serum culture (Table 1).

**Table 1**

| | Cell population cultured from the whole cell population of SVF fraction | Cell population cultured from the CD34-positive & CD45-negative cell population |
|---|---|---|
| CD13 | positive | positive |
| CD31 | negative | negative |
| CD34 | negative | negative |
| CD45 | negative | negative |
| CD90 | positive | positive |
| CD105 | positive | positive |
| CD106 | negative | negative |
| CD117 | negative | negative |

### Example 12

### Transplantation of the pluripotent cells selectively proliferated by low serum culture to an animal model

The pluripotent cells derived from human adipose tissue, divided and grown at an average of 20 times were cultured in a 25 cm² flask coated with human fibronectin, and immediately before a confluent state, the cells were washed with 1 mmol/L of EDTA/ phosphate buffered physiological saline; then 0.25%(w/v) trypsin solution was added, and the mixture was incubated for 2 minutes to peel off the cells. The recovered pluripotent cells (10⁷) were suspended into 200 µl of fibrinogen (Baxter Co.) containing 1 µg of FGF-2 (R&D System Co.). The cell- fibrinogen suspension was injected subcutaneously into the back of a male NOD/SCID mouse (7 weeks old), to which 50 µl of an anti-asialo GM 1 antibody (Wako Pure Chemical Industries Inc.) had been administered intraperitoneally the preceding day.
After a lapse of 2 weeks from the transplantation, a paraffin section was prepared, and HE-stained to observe the state of adipocytes at the transplanted site. As a result, it was confirmed that the transplanted pluripotential stem cells were differentiated into fat (Fig. 10).

## Claims

1. A pluripotent cell comprising the following properties:
(1) the cell is contained in a mixed-cell type population obtained by enzymatic treatment of the tissue collected from an animal;
(2) the cell is contained in a sedimented cell population obtained by centrifugating the mixed-cell type population of the property (1);
(3) the cell selectively proliferates by culturing in a medium containing 2%(v/v) or lower serum and 1 to 100 ng/ml of fibroblast growth factor-2; and
(4) the cell is differentiated into cells having the characteristics of adipocytes, osteoblasts, chondrocytes, tendon cells, myocardial cells, myoblasts, neurocytes or vascular endothelial cells by adjusting the culture condition.

2. The pluripotent cell of claim 1, wherein the animal is human, monkey, mouse, rat, bovine, equine, pig, dog, cat, goat, sheep or chicken.

3. The pluripotent cell of claim 1 or 2, wherein the collected tissue is the subcutaneous adipose, greater omentum, visceral adipose, muscle or organ.

4. The pluripotent cell of claim 1, wherein the cells included in the mixed-cell type population of the property (1) are CD34-positive and CD45-negative cells.

5. The pluripotent cell of claim 4, wherein the selectively proliferating cells of the property (3) are CD34-negative, CD13-positive, CD90-positive and CD105-positive cells.

6. A cell differentiated from the pluripotent cell of claim 1, which has any of the characteristics of adipocytes, osteoblasts, chondrocytes, tendon cells, myocardial cells, myoblast, neurocytes or vascular endothelial cells.

7. A method for obtaining a pluripotent cell having characteristics of adipocytes, osteoblasts, chondrocytes, tendon cells, myocardial cells, myoblast, neurocytes or vascular endothelial cells, which comprises:
(a) a step for preparing a mixed-cell type population by treating an animal tissue with an enzyme;
(b) a step for preparing a sedimented cell population by centrifuging the mixed-cell type population of the step (a); and
(c) a step for selecting a cell selectively proliferating from the cell population of the step (b) by culturing in a medium containing 2%(v/v) or lower serum and 1 to 100 ng/ml of fibroblast growth factor-2.

8. The method according to claim 7, wherein the tissue of human, monkey, mouse, rat, bovine, equine, pig, dog, cat, goat, sheep or chicken is treated with an enzyme to prepare the mixed-cell type population in the step (a).

9. The method according to claim 7, wherein subcutaneous adipose, greater omentum, visceral adipose, muscle or organ in animals is treated with an enzyme to prepare the mixed-cell type population in the step (a).

10. The method according to claim 7, wherein a CD34-positive and CD45-negative cell-type population is prepared in the step (a).

11. The method according to claim 10, wherein a CD34-negative, CD13-positive, CD90-positive and CD105-positive cell is selected in the step (c).

12. A method for cell-transplantation, which comprises transplanting the pluripotent cell of claim 1 into the animal body.
